# EUROPEAN PATENT SPECIFICATION

(11) **EP 2 012 695 B1**
(45) Date of publication and mention of the grant of the patent: **22.07.2015**
(21) Application number: 07754304.9
(22) Date of filing: 28.03.2007
(51) Int. Cl.: A61B 18/14

(54) **ELECTROSURGICAL CUTTING DEVICE**
ELEKTROCHIRURGISCHE SCHNEIDVORRICHTUNG
INSTRUMENT DE COUPE ÉLECTRO-CHIRURGICAL

(30) Priority: 31.03.2006 US 788207 P
(43) Date of publication of application: 14.01.2009
(73) Proprietor: Cook Medical Technologies LLC, Bloomington, IN 47404 (US)
(72) Inventor: KARPIEL, John, A., Winston-Salem, NC 27106 (US)
(74) Representative: Garratt, Peter Douglas
(86) International application number: PCT/US2007/007762
(87) International publication number: WO 2007/126949

(56) References cited:
- WO-A2-00/13602
- US-A1- 2006 064 113

## Description

The invention generally relates to an electrosurgical cutting device for removal of portions of the mucuosa and/or submucosa tissue from the gastrointestinal tract of a human being.

### BACKGROUND

Diagnostic and therapeutic gastrointestinal endoscopy are commonly used to gain access to the digestive tract for the purpose of removing tissue. Common endoscopy procedures include incision and ablation through various known mechanisms.

Techniques for obtaining tissues for biopsies include endoscopic mucosectomy, also known as endoscopic mucosal resection (EMR). Endoscopic mucosectomy involves the removal of a portion (i.e., resection) of the digestive wall including the mucosal membrane. This procedure typically removes a part or even all of the submucosa. Endoscopic mucosectomy is a curative endoscopic procedure which is intended for sessile benign tumors and intramucosal cancers. The procedure makes it possible to determine precisely the nature of any subsequent treatment that may be required.

The incision devices currently utilized in endoscopic mucosectomy make tissue removal difficult. These problems are compounded by the thick gastrointestinal wall that the incisions are performed within. Considerable time and effort is therefore required by the physician to incise and remove the desired tissue. The inability to quickly incise tissue may increase patient trauma. Moreover, current incision devices cannot remove tissue in unfragmented portions. Assessment of fragmented tissue becomes increasingly difficult during sampling as compared to assessment of unfragmented tissue. Furthermore, fragmented resection of early cancers may lead to a higher rate of local tumor recurrence.

In view of the drawbacks of current technology, there is an unmet need for incision devices that can more efficiently remove mucosal and/or submucosal tissue in unfragmented portions in a relatively short period of time without inducing significant patient trauma.

A device according to the preamble of claim 1 is known from WO1999US20045.

### SUMMARY

The invention is defined in claim 1.

Accordingly, an electrosurgical cutting device is provided that resolves or improves upon one or more of the above-described drawbacks.

In a first aspect, an electrosurgical cutting device is disclosed. The device includes a catheter and two or more electrically conductive wires extending through a lumen of the catheter. The wires are movable between a retracted position and an extended cutting position. The wires form a divergent configuration in the extended cutting position.

In a second aspect, a method for performing an EMR procedure is provided. An electrosurgical cutting device comprising a catheter, a plurality of electrically conductive resection wires disposed within a lumen of the catheter, and a handle assembly operably connected to a proximal end of the catheter is provided. Each of the plurality of resection wires comprises a cutting portion. The electrosurgical cutting device is advanced towards a target region having tissue to be incised. Each of the plurality of resection wires extend beyond the distal end of the catheter so as to form a divergent cutting configuration. Each of the plurality of resection wires engages with the target region. Electrical current is applied to the cutting portion of each of the plurality of the resection wires. The handle assembly is manipulated to incise the tissue of the target site and separate the tissue from underlying tissue.

In a third aspect, a method for electrosurgically incising tissue is disclosed. An endoscope is maneuvered towards a target tissue site. An electrosurgical cutting device, which comprises a catheter, multiple electrically conductive cutting wires, and a handle assembly, is advanced into a working channel of an endoscope. Upon reaching the target tissue site, the handle assembly is manipulated in order for the wires to transform from their compressed, retracted configuration to their divergent cutting configuration. Electrical current is applied to the wires to incise tissue from the target tissue site.

### BRIEF DESCRIPTION OF THE DRAWINGS

Embodiments will now be described by way of example with reference to the accompanying drawings, in which:
Figure 1 is a side view of an electrosurgical cutting device with the distal region of the electrically conductive wires extended beyond the distal end of the catheter in a divergent cutting configuration;
Figure 2 is a side view of a hypodermic needle being loaded into a side port of the electrosurgical cutting device;
Figure 3 is a partial cross-sectional view of the distal end of the electrosurgical cutting device with an actuator structure capable of controlling the divergent configuration of the wires;
Figure 4 is a partial cross-sectional view of an electrosurgical cutting device with the wires in a particular extended divergent cutting configuration;
Figure 5 is a partial cross-sectional view of an electrosurgical cutting device with the wires in another particular extended divergent cutting configuration;
Figure 6 is a partial cross-sectional view of an electrosurgical cutting device with the wires retracted within the catheter in a substantially parallel position;
Figure 7 is a partial cross-sectional view of an EMR procedure in which a hypodermic needle is injecting physiological saline solution into a target tissue region;
Figure 8 is a partial cross-sectional view of the cutting device showing a single wire extending within the lumen of the catheter and splitting into two wires at the distal end of the catheter;
Figure 9 is an elevational view of an EMR procedure in which the distal end of the cutting device is positioned within the target tissue;
Figure 10 is an elevational view of an EMR procedure in which the wires are extended within the target tissue site;
Figure 11 is an elevational view of an EMR procedure in which the incision of the target tissue is made and the incised tissue is dragged along the sides of the divergent configuration;
Figure 12 is an elevational view of an EMR procedure in which the wires form a divergent cutting configuration proximal relative to the target tissue site;
Figure 13 is an elevational view of an EMR procedure in which incised tissue enters the pocket of the divergent configuration; and
Figure 14 is a partial cross-sectional view of a snare retrieving the incised tissue.

### DETAILED DESCRIPTION OF THE PREFERRED EMBODIMENTS

The embodiments are described with reference to the drawings in which like elements are referred to by like numerals. The relationship and functioning of the various elements of the embodiments are better understood by the following detailed description. However, the embodiments as described below are by way of example only, and the invention is not limited to the embodiments illustrated in the drawings. It should also be understood that the drawings are not to scale and in certain instances details have been omitted, which are not necessary for an understanding of the embodiments, such as conventional details of fabrication and assembly.

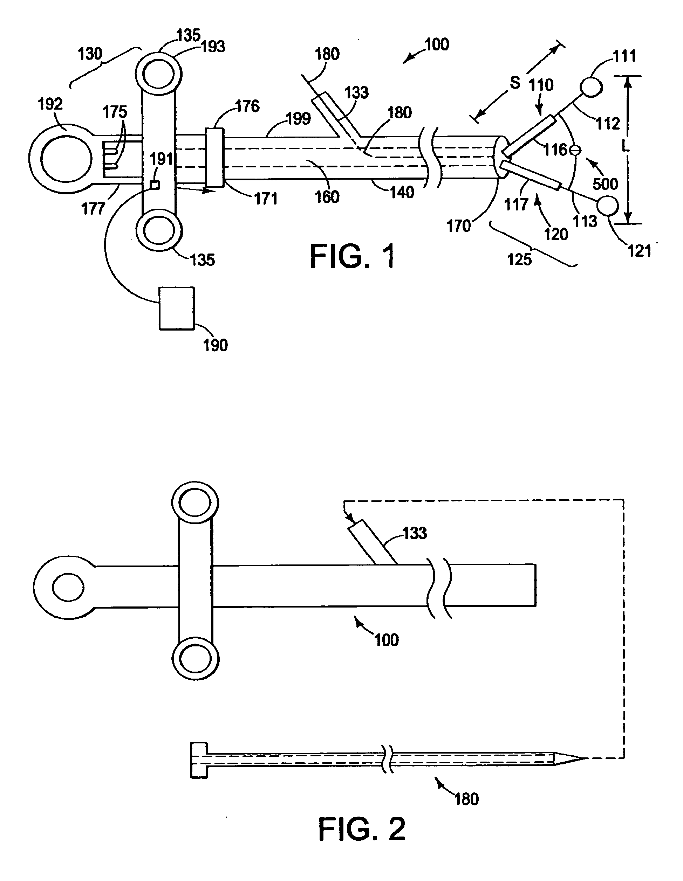

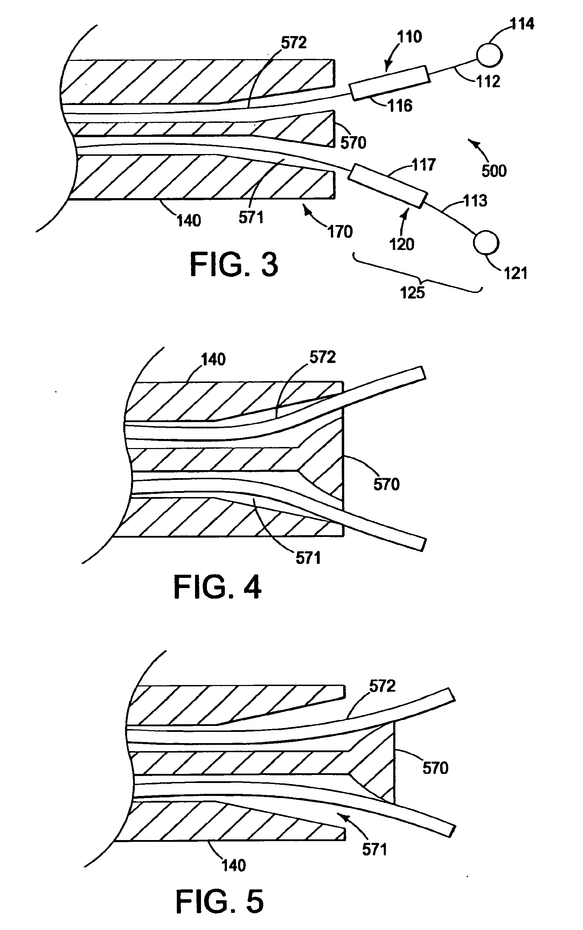

An exemplary electrosurgical cutting device is shown in Figure 1. Figure 1 is a side view showing the electrosurgical cutting device 100 in an extended cutting position. The electrosurgical cutting device 100 includes a catheter 140 having a distal portion 170 and a proximal portion 171, a handle assembly 130 attached to the proximal portion 171 of the catheter 140, electrically conductive wires 110 and 120 extending through a lumen 160 of the catheter 140, and a hypodermic needle 180 movably disposed within the lumen 160 of the catheter 140. The catheter 140 is configured for coupling to an electrosurgical generator 190. The electrosurgical generator 190 is connected to an electrical port 191. The electrosurgical generator 190 supplies the electrical energy to the wires 110 and 120 during the incision of tissue. In general, the incision of the tissue is achieved by extending electrically conductive wires 110 and 120 by a predetermined distance S and angular separation θ. The wires 110 and 120 are movable between a retracted configuration and an extended configuration. The wires 110 and 120 in their extended configuration may flare outwards as shown in Figure 1. Many types of flared shapes are possible. In the example shown in Figure 1, the wires 110 and 120 flare into a divergent configuration 500. The term "divergent" as described herein refers to the wires 110 and 120 separating a predetermined distance from each other in the extended cutting position for at least a portion therealong. Although Figures 1 and 3 show the wires 110 and 120 in their extended cutting position with a V-shaped divergent configuration 500, other variations of the divergent configuration 500 are contemplated. For example, wires 110 and 120 may have a U-shaped divergent configuration or an outward or inward curvature (not shown) near its distal end.

The divergent configuration 500 creates an excise area for receiving tissue within the configuration and/or along the edges of the configuration 500. Electrical current from the electrosurgical generator 190 may be applied as the wires 110 and 120 extend outward into the divergent configuration 500 beyond the distal end 170 of the catheter 140. The current will heat the wires 110 and 120, thereby allowing the tissue to be incised by the wires 110 and 120.

Still referring to Figure 1, handle assembly 130 includes a sliding member 193, a thumb ring 192, and a stem 177. The stem 177 is fixedly attached to the catheter 140, and the sliding member 193 is fixedly attached to the proximal end of the wires 110 and 120. Sliding member 193 is slidably connected to a stem 177, which is affixed to the proximal portion 171 of the catheter 140. Sliding member 193 includes a pair of opposed finger rings 135 which enable a user to grasp the same with the forefinger and index finger. By placing a thumb through thumb ring 192, the sliding member 193 may be pushed and advanced towards the distal end 170 to deploy the electrically conductive wires 110 and 120 beyond the distal end 170 of the catheter 140. Sliding member 193 may also be pulled proximally against stops 175 to retract the distal region 125 of wires 110 and 120 into the distal end 170 of the catheter 140.

An adjustable sliding stop 176 may be disposed over the distal portion 171 of the stem 177. The adjustable sliding stop 176 may be incrementally moved along the proximal portion 171 of catheter 140 to enable controlled extension of the distal region 125 of wires 110 and 120 beyond the distal end 170 of the catheter 140. This facilitates controlled positioning of the distal region 125 of the wires 110 and 120 relative to the distal end 170 of the catheter 140 by a variable distance S. Sliding member 193 has an internally disposed electrical port 191 for making an electrical connection to an electrosurgical generator 190. The electrosurgical generator 190 provides a cutting current as is well known to one of ordinary skill in the art. Although Figure 1 shows the handle assembly 130 and the adjustable sliding stop 176 as the apparatus for activating extension and retraction of the distal region 125 of wires 110 and 120, other actuators may be also be used.

Referring to Figure 2, a hypodermic needle 180 may be inserted into a side port 133 of the electrosurgical cutting device 100. The hypodermic needle 180 may used to inject physiological saline solution into the target tissue. Any type of saline solution known in the art may be used. The saline solution may cause the target tissue to swell and elevate from the underlying normal tissue. Elevation of the target tissue may facilitate removal of the target tissue, such as cancerous tissue or other types of abnormal tissue, during an endoscopic mucosal resection procedure. The ability to remove the target tissue without cutting into it enables a more accurate assessment of the tissue than would otherwise be possible if sampling a fragmented tissue sample. Furthermore, fragmented resection of early cancers may lead to a higher rate of local tumor recurrence. Although Figure 1 shows both the hypodermic needle 180 and the wires 110 and 120 disposed within the lumen 160, electrosurgical cutting device 100 may contain multiple lumens adapted for separately receiving the needle 180 and the wires 110, 120.

Still referring to Figure 1, wires 110 and 120 are shown extending beyond the distal end 170 of the catheter 140 in the predetermined divergent configuration 500. The proximal portions of the wires 110 and 120 are disposed within the lumen 160 of the catheter 140. The distal regions 125 of the wires 110 and 120 are extendable beyond the distal end 170 of the catheter 140. Distal region 125 of wire 110 includes an insulated proximal portion 116, an insulated distal tip 111, and an uninsulated region 112 therebetween. Similarly, distal region 125 of wire 120 includes an insulated proximal portion 117, an insulated distal tip 121, and an uninsulated region 113 therebetween. Insulated tips 111 and 121 prevent burning and injury to deeper layers of tissue surrounding a target site. Ceramic balls may be used as the electrically insulative material for distal tips 111 and 121. Other electrical insulative materials and their respective geometries may be used for the distal tips 111 and 121. Teflon may be used as the electrically insulative material over proximal portions 116 and 117. Other electrical insulative materials may be used for the proximal portions 116 and 117. The uninsulated wire regions 112 and 113 may perform the incision. One skilled in the art may determine suitable lengths of uninsulated regions 112 and 113 indicative of the mucosectomy procedure. A variety of factors can be considered in determining suitable lengths, including the type of mucosectomy to be performed, the size of tissue to be cut and the difficulty of accessing the tissue to be cut.

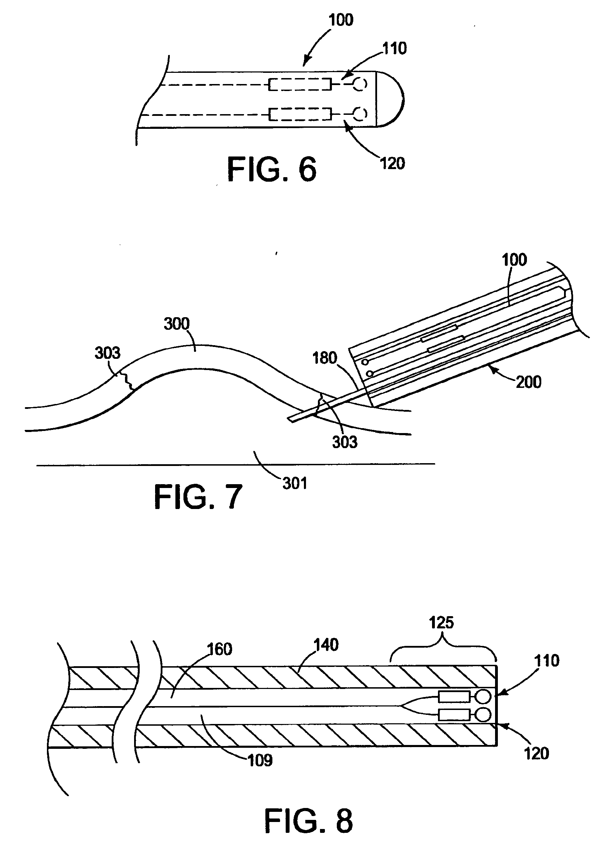

Although the electrosurgical cutting device 100 has been described as having two wires 110, 120, less than or more than two wires may be used. For example, Figure 8 shows that a single wire 109 may extend within the lumen 160 of the catheter 140 and thereafter split into two wires 110, 120 at the distal region 125 of the catheter 140. Figure 8 shows the wires 110 and 120 in their retracted position within the lumen 160. Similar to the example of Figure 1, the wires 110 and 120 of Figure 8 are extendable pass the distal end of the catheter 140 in a flared shape.

The cutting positions of the wires 110 and 120 in their divergent configuration 500 may be further defined by the extension length of distal region 125 beyond the distal end 170 of catheter 140, S, the distance of separation between wires 110 and 120 as measured from the outer edges of distal tips 111 and 121, L, and the angular separation between the wires 110 and 120, θ. Design parameters S, L, and θ may be dependent upon a variety of factors, including the size of tissue desired to be cut and the amount of cancerous tissue present at the target site. Generally, larger values of S, L, and θ may create a larger divergent configuration 500 for incising tissue.

One skilled in the art can determine a suitable diameter of wires 110 and 120. A variety of factors can be considered in determining suitable diameters, including the type of mucosectomy procedure to be performed, the amount of tissue to be cut, and the tendency of the wires to bend at a given S, L, and θ cutting configuration. In this example, the diameter of the wires 110 and 120 may range from about .010 inches to about .020 inches.

Figures 6 and 8 show that wires 110 and 120 may be substantially parallel relative to each other when retracted within the lumen 160 of catheter 140. This is due to the compression wires 110 and 120 undergo when retracted and preloaded within the lumen 160. However, upon the distal region 125 of wires 110 and 120 extending outward from the distal end 170 of the catheter 140, the wires 110 and 120 transform from being substantially parallel to forming a divergent configuration 500.

The divergence of the wires 110 and 120 may be formed by various methods. Heat treatment is one method of biasing the wires 110 and 120 into a divergent configuration 500. The wires 110 and 120 may be heat set by conventional techniques known in the art. The specific heat treatment imparted to set the wires 110 and 120 may determine the natural angular separation between the wires 110 and 120 when the distal regions 125 of wires 110 and 120 expand into their natural divergent configuration 500.

In a preferred embodiment, the wires 110 and 120 may be fabricated from a shape memory metallic alloy such as Nitinol. As is known in the art, shape memory metals undergo a crystalline phase change and thermoelastically deform when heated and cooled. These crystal phase changes are between high temperature austenite and low temperature martensite. Such phase changes enable the wires 110 and 120 to return to their original configuration when cooled. Moreover, the stress-strain behavior of a memory metal alloy makes the material much easier to deform when cooled than at an elevated temperature. The use of Nitinol in this embodiment helps the wires 110 and 120 return to its original orientation if deformed by stress during the cutting procedure. When the wires 110 and 120 are heated by cutting current from electrosurgical generator 190, the crystalline transformation to the austenitic phase makes it much more difficult to deform. If a sufficient force is then applied to the wires 110 and 120 during the procedure, the material can strain to relieve the applied stress as it transforms back to the martensitic phase. Once the stress is reduced, it will unstrain and revert back to austenite. After the applied current is removed, the resultant cooling of the wires 110 and 120 and associated crystal phase change to martensite increases its flexibility. Wires 110 and 120 may be fabricated from other electrically conductive materials, including stainless steel.

The wires 110 and 120 may also extend outwards pass the distal end 170 of the catheter 140 into a divergent configuration 500 by other known methods which do not require any heat treatment. Alternatively, other techniques for biasing the wires 110 and 120 in a divergent configuration may also be utilized. For example, the distal end 170 of the catheter 140 may have structures that guide the wires 110 and 120 along a divergent path such that no heat setting or bending of the wires 110 and 120 is required. By way of a non-limiting example, Figure 3 shows a structure 570 which is disposed at the distal end 170 of the catheter 140. The structure 570 causes the wires 110 and 120 to split into their respective slanted lumens, 572 and 571. Prior to emerging from the distal end 170, the wires 110 and 120 follow the slanted luminal path 572 and 571 as defined by structure 570. Alternatively, divergent lumens at the distal end 170 of the catheter 140 may be used to split the wires 110 and 120 into their divergent configuration 500.

Various types of divergent configuration configurations are possible as illustrated in Figures 4 and 5. The divergent configuration 500 as defined by the extension length of distal region 125 beyond distal end 170 of catheter 140, S, and the distance of separation between wires, L, may be manipulated and controlled by an actuator. The actuator may be structure 570, as shown in Figure 3. The width of the structure 570 may be variably controlled at the proximal end 171 causing the slanted luminal paths 571 and 572 to increase or decrease, thereby altering the divergent configuration 500. Other actuators known to those of ordinary skill in the art are contemplated. The actuator may also be the adjustable sliding stop 176, described in Figure 1.

As described above, Figure 4 shows the electrosurgical cutting device 100 having wires 110 and 120 extended in one possible divergent cutting configuration 500. Figure 5 illustrates electrosurgical cutting device 100 in another possible cutting configuration 500 having a smaller cutting position than that shown in Figure 4. Less movement of the sliding member 193 may be required to generate the cutting position of Figure 5 than that of Figure 4. Various other cutting positions are possible.

Figure 6 is an example of the configuration during advancement of the electrosurgical cutting device 100 shown in Figure 1 to a target incision site. The wires 110 and 120 are shown disposed within the lumen 160 of the catheter 140. The wires 110 and 120 are substantially parallel to each other along the longitudinal length of the lumen 160 of the catheter 140. Figure 8 is another possible configuration during advancement of the device 100. A single wire 109 extends along the length of the catheter 140 within the lumen 160. At the distal region 125, the wire 109 splits into wires 110 and 120.

Wires 110 and 120 may be configured within the catheter 140 such that they cut along a x-y plane or a x-z plane, relative to the handle assembly 130. If wires 110 and 120 cut tissue along the x-y plane, wire 110 may be positioned above wire 120 within catheter 140. If wires 110 and 120 cut tissue along the x-z plane, wire 110 and wire 120 may be positioned side-by-side within the lumen 160 of catheter 140. Determining which configuration to utilize is dependent upon a number of factors, including the shape and size of tissue to be incised.

Catheter 140 is a flexible tubular member. The catheter 140 is formed from any semi-rigid polymer. For example, the catheter 140 can be formed from polyurethane, polyethylene, tetrafluoroethylene, polytetrafluoroethylene, perfluoalkoxl, fluorinated ethylene propylene, or the like. In a typical application, the catheter 140 may have a length of about 220 centimeters in order to sufficiently extend through the working channel of a conventional endoscope. Catheter 140 may also have an outer diameter from about 6 to 7 French in order to fit within the working channel. The catheter 140 may also have a hydrophilic coating 199 overlying its outer surface. The hydrophilic coating 199, when applied to the outer surface of the catheter 140, imparts suppleness and kink resistance to the catheter 140. Hydrophilic coating 199 also provides a highly lubricated surface to facilitate movement through the working channel of the endoscope.

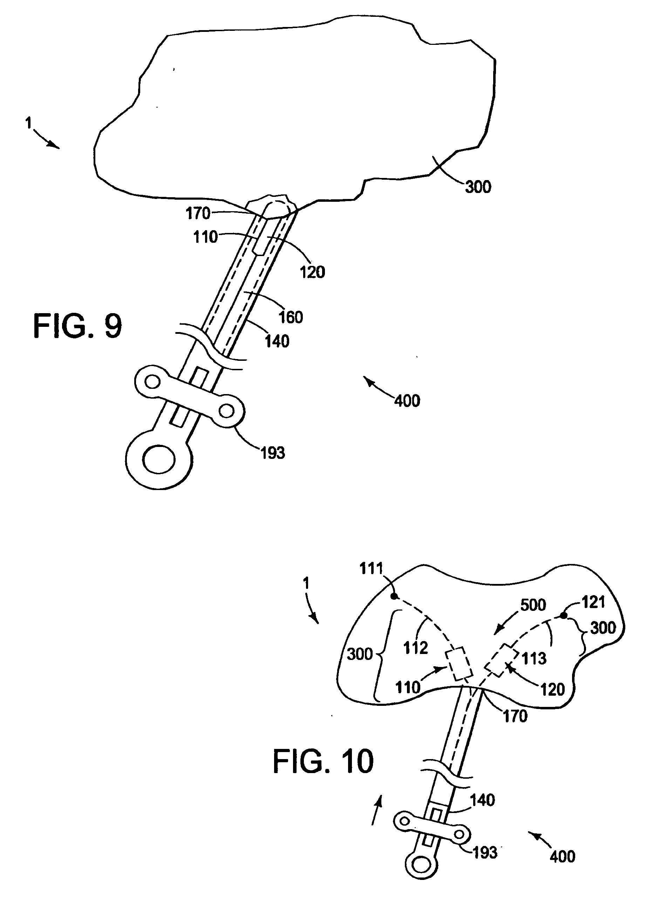

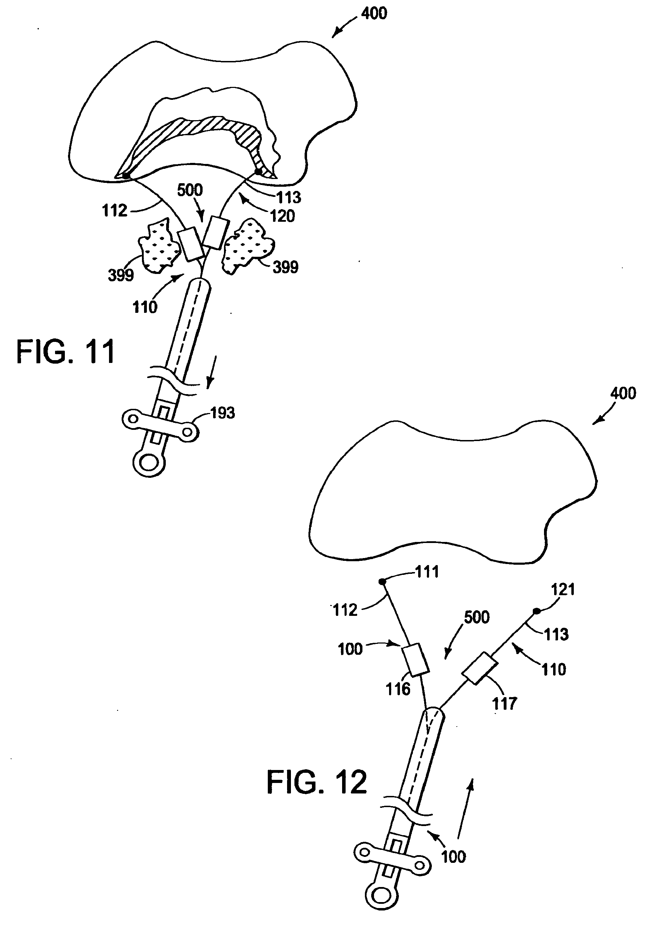

A method of using the electrosurgical cutting device 100 will now be described with reference to Figures 7-14. In particular, and by way of example, Figures 7-14 illustrate a method for performing an Endoscopic Mucosal Resection (EMR) procedure 400. The electrosurgical cutting device 100 may be advanced through a working channel 201 of an endoscope 200 (Figure 7). The device 100 may be maneuvered into a patient down through the esophagus and duodenum, towards the target tissue site 300 within the gastrointestinal lumen 1 (Figures 9, 12). During advancement of the electrosurgical cutting device 100 to a target tissue site 300, the distal regions 125 of the wires 110 and 120 are maintained in a retracted position within the lumen 160 of the catheter 140, as shown in Figure 8. The wires 110 and 120 are maintained in a substantially compressed configuration.

Figure 7 is a partial cross-sectional view of the electrosurgical cutting device 100 in close proximity to the target tissue site 300. After selectively positioning the electrosurgical cutting device 100 in proximity to the target tissue site 300, a physician may examine whether incision markings are needed to define the boundaries of the target tissue site 300. If the margins 303 of the target tissue site 300 are not readily discernible, a typical needle knife tip (not shown) may be loaded into an accessory channel of the endoscope 200 to create markings around the margins 303 of the target tissue 300 to be cut. High frequency current is applied to the tip to create the markings. Such methods for creating markings are well known to those of ordinary skill in the art. Alternatively, markings may be omitted where target tissue site 300 can readily be distinguished from the tissue not intended to be cut, as is the case in Figure 7.

After selectively positioning the electrosurgical cutting device 100 in close proximity to the target tissue site 300, formation of a protrusion of the target tissue site 300 is the next step. The protrusion is created by injecting physiological saline solution through the hypodermic needle 180, which may be disposed within the lumen 181 of the catheter 140. The hypodermic needle 180 may be inserted through the side port 133, as shown in Figure 1. Although not shown in Figure 7, the hypodermic needle 180 may be disposed in the same lumen as the electrosurgical cutting device 100. Figure 7 shows the hypodermic needle 180 extending beyond the distal end 170 of the catheter 140 until it contacts target tissue site 300. A sufficient amount of saline solution, as is known in the art, is injected into the submucosa 301. The submucosa 301 swells, thereby allowing an incision to be made into the target tissue 300 without inadvertently injuring the underlying submucosa tissue 301. After injection of the physiological saline is completed, the hypodermic needle 180 is retracted within the lumen 181. Although the figures illustrate the electrosurgical cutting device 100 as a unitary structure with the hypodermic needle 180, the hypodermic needle 180 may be a separate component that is inserted through a working channel of the endoscope 200.

After the target tissue site 300 has been sufficiently elevated, the process of creating the incision may begin. Figure 9 is an elevated view of the electrosurgical cutting device 100 contacting the target tissue site 300. In particular, the distal end 170 of the catheter 140 is advanced into the target tissue site 300 with the wires 110 and 120 remaining retracted within the lumen 160 of the catheter 140. The wires 110 and 120 may be substantially parallel to each other within the lumen 160, as shown in Figure 9.

After the distal end 170 of the catheter 140 is contained within the target tissue site 300, the sliding member 193 may be advanced distally, as indicated by the arrow in Figure 10, to deploy the wires 110 and 120 beyond the distal end 170 of the catheter 140 and into the tissue of target tissue site 300. As the wires 110 and 120 extend distally and into the target tissue 300, the divergent configuration 500 may be formed. The divergent configuration 500 may open inside the target tissue site 300. The wires 110 and 120 are extended until they make sufficient contact with target tissue site 300 such that uninsulated regions 112 and 113 are disposed distal to a substantial portion of the target tissue site 300 to be incised. Insulated distal tips 111 and 121 are also disposed within target tissue site 300. Adjustable sliding stop 176 (Figure 1) may be moved to a desired position along the proximal region of the catheter 140 to limit the distance S that the wires 110 and 120 may be extended beyond the distal end 170 of the catheter 140. Upon reaching its desired position, sliding stop 176 (Figure 1) may be locked into place. Sliding member 193 may be moved distally in the direction of the arrow until it abuts against adjustable sliding stop 176. This ensures that the distal regions 125 (Figure 1) of wires 110 and 120 are maintained in its divergent configuration 500 at a predetermined distance S during the incision process. Additionally, although not shown, another sliding stop could be placed proximal of the sliding member 193 to prevent retraction of the wires 110 and 120.

Electrical current may be applied from electrosurgical generator 190 (Figure 1) at any time during the procedure to create the incision. The physician sets the current at a setting typically used in such a procedure. Current flows into the electrical port 191 (Figure 1) and thereafter travels through the wires 110 and 120. Uninsulated portions 112 and 113 (Figures 1 and 11) begin to increase in temperature due to resistive heating of these portions of the wires 110 and 120.

As shown in Figure 11, pulling the cutting device 100 in the direction of the arrow (proximally) causes tissue 399 to be incised from target tissue 300. The divergent configuration 500 may be formed within the target tissue site 300 and the divergent configuration 500 may be oriented distally relative to the target tissue site 300. Manipulation of the electrosurgical device 100, either by rotational movement or various other movements, performs the necessary incisions within target tissue 300, as shown in Figure 11. After the incision is made, the tissue 399 may be dragged along the outside of the divergent configuration 500. Figure 11 shows incised tissue 399 dragged along the outside of the configuration of the 500. The extension length of the distal region 125 beyond the distal end 170 of the catheter 140, S, the distance of separation between the wires 110 and 120 as measured from the outer edges of distal tips 111 and 121, L, and the angular separation θ between the wires 110 and 120 (Figure 1) will determine the amount of tissue 399 that is incised. In this example, a length of about three centimeters of tissue 399 from target tissue site 300 is incised.

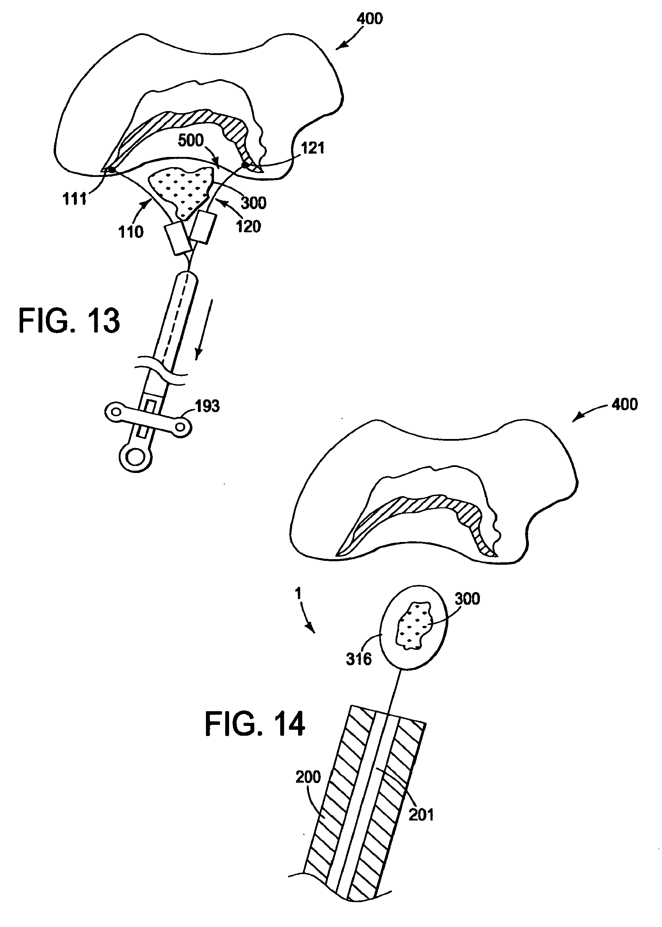

Alternatively, Figures 12 and 13 show another possible method of using the electrosurgical cutting device 100 to incise tissue. Unlike the method described in Figures 9-11, the distal end 170 of the catheter 140 may be advanced toward the target tissue site 300 with the wires 110 and 120 already extending distally past the distal end 170 of the catheter 140. This forms the divergent configuration 500 prior to the device 100 entering the target tissue site 300. In other words, the divergent configuration 500 is oriented proximal relative to the target tissue site 300, as shown in Figure 12. With the wires 110 and 120 extended in their predetermined position to form the divergent configuration 500, the physician pushes the electrosurgical cutting device 100 towards target tissue site 300 in the direction indicated by the arrow (distally) of Figure 12. As shown in Figure 13, uninsulated portions 112 and 113 begin to incise the target tissue site 300. Pushing the electrosurgical device 100 forward causes the target tissue site 300 to be incised and thereafter enter the interior of the divergent configuration 500. Electrical current may be applied from electrosurgical generator 190 (Figure 1) at any time during the procedure to create the incision.

Pulling the wires 110 and 120 in the proximal direction as indicated by the arrow in Figure 13 causes the incised tissue from the target tissue site 300 to enter the divergent configuration 500. Similar to the cutting configuration described in Figures 9-11, the extension length of the distal region 125 beyond the distal end 170 of the catheter 140, S, the distance of separation between the wires 110 and 120 as measured from the outer edges of distal tips 111 and 121, L, and the angular separation, θ, between the wires 110 and 120 (Figure 1) may determine the amount of tissue 300 that is incised.

After the target tissue 300 has been incised, the handle assembly 130 may be pulled to withdraw the electrosurgical cutting device 100 through the endoscope 200. A retrieval device, such as a snare 316 or forceps (not shown), may subsequently be used to remove the incised target tissue 300 through a working channel 201 of the endoscope 200, as shown in Figure 14.

Although not shown in the Figures, the wires 110 and 120 of the electrosurgical cutting device 100 may also form a particular divergent configuration 500 during the procedure 400 that causes some of the incised tissue to enter the divergent configuration 500 and some of the incised tissue to drag along the outside of the divergent configuration 500.

In an alternative embodiment, the electrosurgical cutting device 100 may be used in a non-EMR procedure. In such a procedure, because physiological saline solution may not be required to lift the target tissue from the underlying normal tissue, hypodermic needle 180 (Figure 1) may be removed from the lumen 160 of the catheter 140. With the exception of no saline required to be injected to elevate the target tissue from the underlying normal tissue, the method for incising tissue is substantially similar to that described above with reference to Figures 9-14.

The above figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims.

## Claims

1. An electrosurgical cutting device comprising:
a catheter (140) having a proximal end (171), a distal end (170), and one or more lumens extending from the proximal end to the distal end of the catheter, the catheter being configured for coupling to an electrosurgical generator; and
a plurality of electrically conductive cutting wires (110, 120) disposed within the one or more lumens movable between a retracted position and an extended cutting position, wherein in the extended cutting position a distal region of the wires extends outwardly from the distal end of the catheter to form a divergent cutting configuration (500);
**characterised in that**:
a structure (570) is disposed at the distal end of the catheter causing the wires to split into respective slanted lumens (572, 571);
wherein the structure (570) is adapted to be variably controlled at the proximal end of the catheter causing the slanted luminal paths to increase or decrease thereby altering the divergent configuration (500).

2. The electrosurgical cutting device of claim 1, further comprising a handle operably connected to the proximal end of the catheter, wherein the handle is configured to manipulate the electrically conductive cutting wires between the retracted position and the extended cutting position.

3. The electrosurgical cutting device of claim 2, wherein the handle comprises a sliding member slidably disposed on a stem.

4. The electrosurgical device of claim 2, wherein the handle is coupled to an electrosurgical generator for providing electrical current to the wires.

5. The electrosurgical cutting device of any preceding claim, further comprising a hypodermic needle extendable from the proximal end to the distal end of the catheter.

6. The electrosurgical cutting device of any preceding claim, wherein each of the plurality of the electrically conductive wires, when in the retracted position, is positioned within the distal end of the catheter and substantially parallel to each other.

7. The electrosurgical device of any preceding claim, wherein each of the plurality of wires further comprises an uninsulated portion between a proximal portion and a distal tip, the proximal portion and the distal tip being insulated, the uninsulated portion being adapted for incising tissue.

8. The electrosurgical device of claim 7, wherein the distal tip of each of the plurality of the wires is insulated with ceramic.

9. The electrosurgical device of any preceding claim, wherein the proximal portion of the distal region of each of the plurality of wires is insulated with polytetrafluoroethylene.

10. The electrosurgical device of any preceding claim, wherein each of the plurality of the wires is formed from a metallic alloy.

11. The electrosurgical device of any preceding claim, wherein the catheter has a length of about 150 centimeters to about 220 centimeters and a diameter ranging from about 6 French to about 7 French.

12. The electrosurgical device of any preceding claim, wherein the distal region of each of the plurality of wires is insulated at a proximal portion and at a distal tip.

## Patentansprüche

1. Elektrochirurgische Schneidvorrichtung, umfassend:
einen Katheter (140) mit einem proximalen Ende (171), einem distalen Ende (170) und einem oder mehreren Lumen, die sich von dem proximalen Ende zu dem distalen Ende des Katheters erstrecken, wobei der Katheter zur Verbindung mit einem elektrochirurgischen Generator ausgelegt ist; und
eine Vielzahl von elektrisch leitenden Schneiddrähten (110, 120), die in dem einen oder den mehreren Lumen angeordnet sind und zwischen einer zurückgezogenen Stellung und einer ausgezogenen Schneidestellung bewegbar sind, worin sich in der ausgezogenen Schneidestellung eine distale Region der Drähte von dem distalen Ende des Katheters nach außen erstreckt, um eine divergente Schneidkonfiguration (500) zu bilden;
**dadurch gekennzeichnet, dass**:
eine Konstruktion (570) an dem distalen Ende des Katheters angeordnet ist, die verursacht, dass sich die Drähte in jeweilige schräge Lumen (572, 571) teilen; worin die Konstruktion (570) ausgelegt ist, an dem proximalen Ende des Katheters variabel gesteuert zu werden, wodurch die schrägen Lumenpfade zunehmen oder abnehmen und so die divergente Konfiguration (500) verändert wird.

2. Elektrochirurgische Schneidvorrichtung nach Anspruch 1, ferner einen Griff umfassend, der mit dem proximalen Ende des Katheters in Wirkverbindung steht, worin der Griff ausgelegt ist, die elektrisch leitenden Schneiddrähte zwischen der zurückgezogenen Stellung und der ausgezogenen Schneidestellung zu manipulieren.

3. Elektrochirurgische Schneidvorrichtung nach Anspruch 2, worin der Griff ein Gleitelement umfasst, das gleitend auf einem Schaft angeordnet ist.

4. Elektrochirurgische Schneidvorrichtung nach Anspruch 2, worin der Griff zur Zuführung von elektrischem Strom zu den Drähten mit einem elektrochirurgischen Generator verbunden ist.

5. Elektrochirurgische Schneidvorrichtung nach einem der vorhergehenden Ansprüche, ferner eine Injektionskanüle umfassend, die von dem proximalen Ende zu dem distalen Ende des Katheters ausgezogen werden kann.

6. Elektrochirurgische Schneidvorrichtung nach einem der vorhergehenden Ansprüche, worin jeder der Vielzahl von elektrisch leitenden Drähten in der zurückgezogenen Stellung in dem distalen Ende des Katheters und im Wesentlichen parallel zueinander angeordnet ist.

7. Elektrochirurgische Schneidvorrichtung nach einem der vorhergehenden Ansprüche, worin jeder der Vielzahl von Drähten ferner einen nicht isolierten Abschnitt zwischen einem proximalen Abschnitt und einer distalen Spitze umfasst, wobei der proximale Abschnitt und die distale Spitze isoliert sind, wobei der nicht isolierte Abschnitt zum Einschneiden in Gewebe ausgelegt ist.

8. Elektrochirurgische Schneidvorrichtung nach Anspruch 7, worin die distale Spitze jedes der Vielzahl von Drähten mit Keramik isoliert ist.

9. Elektrochirurgische Schneidvorrichtung nach einem der vorhergehenden Ansprüche, worin der proximale Abschnitt der distalen Region jedes der Vielzahl von Drähten mit Polytetrafluorethylen isoliert ist.

10. Elektrochirurgische Schneidvorrichtung nach einem der vorhergehenden Ansprüche, worin jeder der Vielzahl von Drähten aus einer Metalllegierung geformt ist.

11. Elektrochirurgische Schneidvorrichtung nach einem der vorhergehenden Ansprüche, worin der Katheter eine Länge von ungefähr 150 cm bis ungefähr 220 cm und einen Durchmesser im Bereich von ungefähr 6 French bis ungefähr 7 French aufweist.

12. Elektrochirurgische Schneidvorrichtung nach einem der vorhergehenden Ansprüche, worin die distale Region jedes der Vielzahl von Drähten an einem proximalen Abschnitt und an einer distalen Spitze isoliert ist.

## Revendications

1. Instrument de coupe électro-chirurgical comportant :
un cathéter (140) présentant une extrémité (171) proximale, une extrémité (170) distale, et une ou plusieurs lumières s'étendant de l'extrémité proximale à l'extrémité distale du cathéter, le cathéter étant configuré pour être accouplé à un générateur électro-chirurgical ; et
une pluralité de fils métalliques (110, 120) de coupe électro-conducteurs disposés à l'intérieur desdites une ou plusieurs lumières mobiles entre une position rétractée et une position déployée de coupe, dans lequel, dans la position déployée de coupe, une région distale des fils métalliques s'étend vers l'extérieur depuis l'extrémité distale du cathéter pour former une configuration (500) de coupe divergente ;
**caractérisé en ce que** :
une structure (570) disposée au niveau de l'extrémité distale du cathéter amène les fils métalliques à se séparer en lumières (572, 571) obliques respectives ;
dans lequel la structure (570) est conçue pour être commandée de manière variable au niveau de l'extrémité proximale du cathéter, amenant les voies luminales obliques à augmenter ou à diminuer, modifiant ainsi la configuration (500) divergente.

2. Instrument de coupe électro-chirurgical selon la revendication 1, comportant en outre une poignée reliée de manière fonctionnelle à l'extrémité proximale du cathéter, dans lequel la poignée est configurée pour manipuler les fils métalliques de coupe électro-conducteurs entre la position rétractée et la position déployée de coupe.

3. Instrument de coupe électro-chirurgical selon la revendication 2, dans lequel la poignée comporte un élément coulissant disposé à coulissement sur une tige.

4. Instrument électro-chirurgical selon la revendication 2, dans lequel la poignée est accouplée à un générateur électro-chirurgical destiné à fournir un courant électrique aux fils métalliques.

5. Instrument de coupe électro-chirurgical selon l'une quelconque des revendications précédentes, comportant en outre une aiguille hypodermique pouvant s'étendre de l'extrémité proximale à l'extrémité distale du cathéter.

6. Instrument de coupe électro-chirurgical selon l'une quelconque des revendications précédentes, dans lequel chaque fil métallique de la pluralité de fils métalliques électro-conducteurs, lorsque dans la position rétractée, est positionné à l'intérieur de l'extrémité distale du cathéter et sensiblement parallèle aux autres.

7. Instrument électro-chirurgical selon l'une quelconque des revendications précédentes, dans lequel chaque fil métallique de la pluralité de fils métalliques comporte en outre une partie non isolée entre une partie proximale et une pointe distale, la partie proximale et la pointe distale étant isolées, la partie non isolée étant conçue pour inciser du tissu.

8. Instrument électro-chirurgical selon la revendication 7, dans lequel la pointe distale de chaque fil métallique de la pluralité de fils métalliques est isolée avec de la céramique.

9. Instrument électro-chirurgical selon l'une quelconque des revendications précédentes, dans lequel la partie proximale de la région distale de chaque fil métallique de la pluralité de fils métalliques est isolée avec du polytétrafluoroéthylène.

10. Instrument électro-chirurgical selon l'une quelconque des revendications précédentes, dans lequel chaque fil métallique de la pluralité de fils métalliques est formé à partir d'un alliage métallique.

11. Instrument électro-chirurgical selon l'une quelconque des revendications précédentes, dans lequel le cathéter a une longueur d'environ 150 à environ 220 centimètres et un diamètre compris entre environ 6 à environ 7 French.

12. Instrument électro-chirurgical selon l'une quelconque des revendications précédentes, dans lequel la région distale de chaque fil métallique de la pluralité de fils métalliques est isolée au niveau d'une partie proximale et au niveau d'une pointe distale.
